(19) Europäisches Patentamt
European Patent Office
Office européen des brevets

(11) **EP 3 566 651 B1**

(12) **EUROPÄISCHE PATENTSCHRIFT**

(45) Veröffentlichungstag und Bekanntmachung des Hinweises auf die Patenterteilung:
**29.06.2022   Patentblatt 2022/26**

(21) Anmeldenummer: **18171283.7**

(22) Anmeldetag: **08.05.2018**

(51) Internationale Patentklassifikation (IPC):
**A61B 6/03** (2006.01)    **A61B 6/00** (2006.01)
**G06T 7/00** (2017.01)

(52) Gemeinsame Patentklassifikation (CPC):
**A61B 6/032; A61B 5/1071; A61B 5/1072; A61B 5/4585; A61B 5/7264; A61B 6/505; G06T 7/0012; G06T 7/60; G16H 30/40; G16H 50/30;** A61B 5/055; G06T 2207/10081; G06T 2207/10088; G06T 2207/10116; G06T 2207/10132;    (Forts.)

(54) **VERFAHREN UND VORRICHTUNG ZUR ERMITTLUNG VON ERGEBNISWERTEN AUF BASIS EINER SKELETTALEN MEDIZINTECHNISCHEN BILDAUFNAHME**

METHOD AND DEVICE FOR DETERMINING RESULT VALUES BASED ON A SKELETAL MEDICAL IMAGE CAPTURE

PROCÉDÉ ET DISPOSITIF DE DÉTERMINATION DES VALEURS DE RÉSULTAT À BASE D'UNE IMAGE MÉDICO-TECHNIQUE SQUELETTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**13.11.2019   Patentblatt 2019/46**

(73) Patentinhaber: **Siemens Healthcare GmbH 91052 Erlangen (DE)**

(72) Erfinder: **Fieselmann, Andreas 91052 Erlangen (DE)**

(56) Entgegenhaltungen:
WO-A1-2006/000063    DE-A1-102007 007 803
US-A1- 2017 196 526    US-A1- 2018 061 090

• **CHI-WEN HSIEH ET AL: "Bone age estimation based on phalanx information with fuzzy constrain of carpals", MEDICAL & BIOLOGICAL ENGINEERING & COMPUTING, SPRINGER, BERLIN, DE, Bd. 45, Nr. 3, 23. Januar 2007 (2007-01-23), Seiten 283-295, XP019864828, ISSN: 1741-0444, DOI: 10.1007/S11517-006-0155-9**

EP 3 566 651 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
G06T 2207/30008

**Beschreibung**

Die Erfindung betrifft ein Verfahren und eine Vorrichtung zur Ermittlung von Ergebniswerten auf Basis einer skelettalen medizintechnischen Bildaufnahme. Die Erfindung betrifft des Weiteren eine entsprechende Befundungsstation zur Befundung einer skelettalen medizintechnischen Bildaufnahme sowie ein entsprechendes medizintechnisches bildgebendes System.

In der (muskulo)skelettalen Radiologie werden häufig Längenund Winkelmessungen an Knochen durchgeführt. Beispielsweise werden im Kniegelenk verschiedene Abstände und Winkel von Patella, Femur und Tibia vermessen, um dort Fehlstellungen zu diagnostizieren, geeignete Therapiemaßnahmen zu bestimmen oder einen Therapieverlauf zu kontrollieren. In der Regel wird bei diesen Messungen wie folgt vorgegangen:

Zunächst erfolgt das Vermessen der betreffenden Bilder durch einen Befunder, z.B. einen Arzt. In diesem Rahmen werden bestimmte Längen und Winkel in den Bildern vermessen und aus diesen Maßen Parameter berechnet, wie z.B. ein Verhältnis zweier Längen. Ein solcher Parameter wird im Folgenden als "Orthopädischer Ergebniswert" bezeichnet. Die Längen und Winkel ergeben sich aus Referenzpunkten in den Bildern, die auch als (anatomische) "Landmarken" (engl.: "Landmarks") bezeichnet werden. Die Vermessung erfolgt in der Praxis manuell und bei digitalen Bildern in der Regel unterstützt durch die Messwerkzeuge (z.B. eine Abstandsmessfunktion) einer Bildbefundungssoftware.

Nach der Vermessung erfolgt eine Klassifizierung der Messergebnisse mittels vorher festgelegter Normbereiche. Diese Normbereiche werden dabei mit dem bei der Vermessung ermittelten orthopädischen Ergebniswert verglichen und danach entschieden, in welche Klassifikation der vermessene Bildausschnitt einzuordnen ist.

Nach der Klassifizierung erfolgt die Erstellung eines Berichtes bzw. einer Dokumentation der Messergebnisse und der Klassifikationen.

US 2017/196526 A1 offenbart ein Verfahren zur automatischen Ermittlung einer Belastungsinformation eines Gelenks eines Patienten, wobei Bilddatensätze des belasteten Gelenks mittels einer Bildaufnahmeeinrichtung aufgenommen wird, dadurch gekennzeichnet, dass mehrere Bilddatensätze, von denen einer dreidimensional ist, jeweils für unterschiedliche Belastungszustände aufgenommen werden und durch gemeinsame Auswertung die Gelenkbelastungsinformation ermittelt wird.

WO 2006/000063 A1 beschreibt ein Verfahren zum Durchführen einer kephalometrischen oder anthropometrischen Analyse, umfassend die Schritte Erfassen eines 3D-Scans des Kopfes einer Person unter Verwendung einer medizinischen 3D-Bildmodalität, Erzeugen eines 3D-Oberflächenmodells unter Verwendung von Daten aus dem 3D-Scan, Erzeugen von mindestens einem 2D-Cephalogramm, das geometrisch mit dem 3D-Oberflächenmodell verknüpft ist, aus dem 3D-Scan, Angeben anatomischer Landmarken auf mindestens einem 2D-Cephalogramm bzw. auf dem 3D-Oberflächenmodell, Durchführen der Analyse unter Verwendung der anatomischen Landmarken.

Ein gravierender Nachteil des bekannten Vorgehens ist, dass Ärzte wie z.B. Radiologen, Orthopäden oder Chirurgen die Messungen manuell durchführen müssen. Für den Arzt ist eine solche manuelle Vorgehensweise zeitaufwändig und oft auch unbeliebt. Zudem nimmt eine manuelle Vorgehensweise eine nicht unbeträchtliche Zeit in Anspruch, was sich nicht zuletzt für einen Patienten negativ auf die Kosten einer Untersuchung auswirkt.

Ein weiterer Nachteil einer manuellen Vermessung und Auswertung ist, dass die oben genannten Normbereiche von unterschiedlichen Faktoren abhängen, z.B. sind die Normbereiche altersabhängig. Damit muss bei einer manuellen Herangehensweise oftmals vom Befunder eine externe Referenz zum Nachschlagen der Normbereiche in Anspruch genommen werden, was zu Fehlern führen kann, die z.B. aus einer falschen Ablesung oder Eintragung von Werten resultieren.

Zudem wirkt sich bei einer Verlaufskontrolle einer Therapie, bei der üblicherweise mehrere Aufnahmen über einen bestimmten Zeitraum aufgenommen werden, negativ aus, wenn verschiedene Aufnahmen von unterschiedlichen Ärzten durchgeführt und ausgewertet werden. Aufgrund der manuellen Auswertung können die Messergebnisse subjektiv beeinflusst sein, was zu einer Erschwerung der Interpretation der Messergebnisse führen kann, wie z.B. das Erkennen einer Veränderung über einen bestimmten Zeitraum.

Es ist eine Aufgabe der vorliegenden Erfindung, ein alternatives, komfortableres Verfahren bzw. eine entsprechende Vorrichtung zur Ermittlung von Ergebniswerten auf Basis einer skelettalen medizintechnischen Bildaufnahme, sowie eine entsprechende Befundungsstation zur Befundung einer skelettalen medizintechnischen Bildaufnahme und ein entsprechendes medizintechnisches bildgebendes System anzugeben, mit dem die oben beschriebenen Nachteile vermieden werden.

Diese Aufgabe wird durch ein Verfahren gemäß Patentanspruch 1, eine Vorrichtung gemäß Patentanspruch 10 sowie durch eine Befundungsstation gemäß Patentanspruch 12 und ein medizintechnisches bildgebendes System gemäß Patentanspruch 13 gelöst.

Das erfindungsgemäße Verfahren und die erfindungsgemäße Vorrichtung dienen zur Ermittlung von Ergebniswerten auf Basis einer skelettalen medizintechnischen Bildaufnahme, insbesondere zu einer automatisierten Messung und ggf. auch zu einer Klassifikation und/oder einer möglichen Visualisierung des Einflusses der Positionierung der Messpunkte auf die Ergebniswerte. Solche Bildaufnahmen können sowohl Projektionsbilder sein, z.B. aufgenommen mittels einem Radiographiesystem, als auch tomographische Bilddaten, z.B. aufgenommen mittels CT, MRT, Cone-Beam CT oder Ultraschall. Im Grunde ist es unerheblich, wie die Bildaufnahmen angefertigt wurde, so lange die für eine Untersuchung relevanten Bereiche zu erkennen sind. Die Ergebniswerte werden anhand von Daten ermittelt, die sich aus den Bildaufnahmen ergeben, insbesondere direkt aus den Bilddaten (z.B. bei digitalen Bildaufnahmen) oder aus Daten, die sich aus einem Koordinatensystem ergeben, welches über die Bildaufnahme projiziert wird (z.B. bei analogen Bildaufnahmen).

Das erfindungsgemäße Verfahren umfasst die Schritte:

- Bereitstellung einer Bildaufnahme
  In diesem Schritt wird eine skelettale medizintechnische Bildaufnahme bereitgestellt. Dies kann dadurch erfolgen, dass eine Bildaufnahme für das Verfahren angefertigt wird, also durch ein Bildaufnahmesystem aufgenommen und ggf. die Bildaufnahme aus Rohdaten rekonstruiert wird. Die Bildaufnahme kann aber auch älter sein und z.B. durch ein Radiologieinformationssystem ("RIS") aus einem PACS (Picture Archiving and Communication System = Bildarchivierungs- und Kommunikationssystem) zur Verfügung gestellt werden. Die Bildaufnahmen können dabei zweidimensional oder dreidimensional sein. Die Bereitstellung und die im Folgenden beschriebenen Schritte können auch im Rahmen des "Cloud Computing" erfolgen, wie im weiteren noch ausführlicher dargestellt wird.

- Bestimmung von Referenzpunkten
  In diesem Schritt erfolgt eine automatische Bestimmung von Referenzpunkten in der Bildaufnahme. Gemäß einem Aspekt der Erfindung kann eine automatische Bestimmung vorzugsweise mittels eines Algorithmus erreicht werden, der auf den Prinzipien des maschinellen Lernens basiert. Dies geschieht besonders bevorzugt mittels einer erfindungsgemäßen Vorrichtung wie sie weiter unten beschrieben wird. Mit der Bestimmung der Referenzpunkte sind deren Koordinaten in der Bildaufnahme (z.B. Pixelpositionen im Bild oder in einem auf das Bild projiziertes Koordinatensystem) dem Verfahren bekannt.

- Berechnung eines Ergebniswerts
  In diesem Schritt wird ein orthopädischer Ergebniswert berechnet. Der Begriff "orthopädischer Ergebniswert" impliziert dabei, dass dieser orthopädisch relevante Werte wiedergibt, die Ergebnis der durchgeführten Berechnung sind. Die Berechnung basiert zumindest auf einem Abstand zwischen zwei Referenzpunkten und/oder auf einem von Referenzpunkten definierten Winkel. Der Winkel kann beispielsweise als Winkel in einem Dreieck definiert sein, welches durch die drei Referenzpunkte aufgespannt wird, mit einem der Referenzpunkte als Scheitelpunkt des Winkels. Der Winkel kann aber beispielsweise auch über vier Referenzpunkte definiert werden, wobei jeweils zwei Punkte eine Gerade definieren, die sich in einem Winkel schneiden.

Mittels der bekannten Koordinaten der Referenzpunkte (siehe vorangehenden Schritt) werden Abstände bzw. Winkel berechnet. Anschließend kann beispielsweise als ein Ergebniswert aus den Abständen ein Abstandsverhältnis ermittelt werden.

Die erfindungsgemäße Vorrichtung umfasst:

- Eine Datenschnittstelle zur Erfassung von Bilddaten einer skelettalen medizintechnischen Bildaufnahme. Dies kann sowohl eine normale Datenschnittstelle im Rahmen eines RIS oder PACS sein, als auch eine Datenschnittstelle (z.B. ein Datenbus) in einem medizintechnischen bildgebenden System, die z.B. direkt Bilddaten einer Bildrekonstruktionseinheit oder eines Bilddetektors erfasst. Diese Datenschnittstelle kann aber auch Bilddaten eines Scanners erfassen, über den analoge Bildaufnahmen eingescannt werden können. Durch eine digitale Übermittlung einer Bildaufnahme steht automatisch ein Koordinatensystem zur Verfügung, nämlich die Position der Pixel in der Bildaufnahme. Dieses Koordinatensystem kann ggf. durch eine Transformationseinheit transformiert werden, z.B. auf die wahre Größe der aufgenommenen Region oder in Raumkoordinaten des Patienten.

- Eine Referenzpunkt-Bestimmungseinheit ausgelegt für eine automatische Bestimmung von Referenzpunkten in der Bildaufnahme. Die Referenzpunkt-Bestimmungseinheit umfasst dazu bevorzugt einen im Rahmen des maschinellen Lernens trainierten Algorithmus, bevorzugt ein neuronales Netzwerk. Die automatische Bestimmung von Referenzpunkten in der Bildaufnahme kann dann mittels dieses Algorithmus erfolgen.

- Eine Ergebniswert-Ermittlungseinheit ausgelegt für eine Berechnung eines orthopädischen Ergebniswerts basierend zumindest auf einem Abstand zwischen zwei Referenzpunkten und/oder auf einem von Referenzpunkten definierten Winkel (s.o.).

Eine erfindungsgemäße Befundungsstation zur Befundung einer skelettalen medizintechnischen Bildaufnahme ist zur Durchführung eines erfindungsgemäßen Verfahrens ausgestaltet. Alternativ oder Ergänzend umfasst sie eine erfindungsgemäße Vorrichtung. Die Befundungsstation kann im Rahmen eines RIS mit einer medizintechnischen bildgebenden Aufnahmeeinheit oder einem PACS datentechnisch verbunden sein oder sie ist zumindest für eine Integration in ein RIS bzw. für eine datentechnische Verbindung mit einer medizintechnischen bildgebenden Aufnahmeeinheit oder einem PACS ausgestaltet.

Ein erfindungsgemäßes medizintechnisches bildgebendes System umfasst eine erfindungsgemäße Vorrichtung, insbesondere eine erfindungsgemäße Befundungsstation.

Ein Großteil der zuvor genannten Komponenten der Vorrichtung bzw. der Befundungsstation, können ganz oder teilweise in Form von Softwaremodulen in einem Prozessor einer entsprechenden Vorrichtung bzw. Befundungsstation realisiert werden. Eine weitgehend softwaremäßige Realisierung hat den Vorteil, dass auch schon bisher verwendete Vorrichtungen bzw. Befundungsstationen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung einer Befundungsstation ladbar ist, mit Programmabschnitten, um alle Schritte des erfindungsgemäßen Verfahrens auszuführen, wenn das Programm in dem Rechensystem bzw. der Befundungsstation ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zum Rechensystem bzw. zur Befundungsstation und/oder zur Speicherung an oder in dem Rechensystem bzw. der Befundungsstation kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der Befundungsstation einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Wie oben bereits angemerkt wurde, kann das Verfahren auch Elemente des "Cloud Computing" (in Deutsch etwa: Berechnung in einer "Datenwolke") umfassen. Beim "Cloud Computing" wird eine IT-Infrastruktur, z.B. Speicherplatz oder Rechenleistung aber auch Anwendungssoftware über ein Netzwerk zur Verfügung gestellt. Die Kommunikation zwischen dem Anwender und der "Cloud" erfolgt dabei mittels Datenschnittstellen und Datenübertragungsprotokollen.

Im Rahmen des "Cloud Computings" erfolgt bei einer bevorzugten Ausführungsform des erfindungsgemäßen Verfahrens eine Bereitstellung einer skelettalen medizintechnischen Bildaufnahme über einen Datenkanal (z.B. ein Netzwerk) an eine "Cloud". Diese "Cloud" umfasst ein (Remote-) Rechensystem, z.B. einen Computercluster, das in der Regel nicht den Rechner des Benutzers umfasst. Diese Cloud kann insbesondere durch die medizinische Einrichtung, die auch die medizintechnischen bildgebenden Systeme bereitstellt, zur Verfügung gestellt werden. Insbesondere werden die Daten der Bildaufnahme über ein RIS oder PACS an ein (Remote-) Rechnersystem (die "Cloud") gesendet. Bevorzugt stellen das Rechensystem der "Cloud", das Netzwerk sowie das medizintechnische bildgebende System einen Verbund im datentechnischen Sinne dar. Das Verfahren kann dabei mittels einer Befehlskonstellation in dem Netzwerk realisiert werden.

Die automatische Bestimmung von Referenzpunkten erfolgt bevorzugt in der "Cloud". Bevorzugt kann der Anwender jedoch bereits eine Bearbeitung der Bildaufnahme auf seinem lokalen Rechner (auch im Rahmen des erfindungsgemäßen Verfahrens) vorgenommen haben, z.B. indem er in einer Vorabdarstellung bereits (ggf. grob) einige Referenzpunkte eingetragen hat oder berechnen ließ.

Eine Berechnung eines orthopädischen Ergebniswerts erfolgt ebenfalls bevorzugt in der "Cloud", obwohl sie je nach Anwendungsfall und Komplexität der Berechnung auch auf dem lokalen Rechner des Anwenders erfolgen kann (z.B. im Rahmen der vorgenannten Vorabbearbeitung oder auf Basis von durch die Cloud mitgeteilten Abständen/Winkeln)). Zumindest werden in der "Cloud" vorzugsweise benötigte Abstände und/oder Winkel bestimmt.

Die in der Cloud berechneten Daten oder Ergebnisse dieser Daten werden bevorzugt wieder über einen Datenkanal (z.B. ein Netzwerk) zu dem lokalen Rechner des Anwenders gesendet. In dem oben angesprochenen Beispiel werden die Daten der bereitgestellten Bildaufnahme also durch ein Rechnersystem eines Krankenhauses gemäß des erfindungsgemäßen Verfahrens bearbeitet und die Ergebnisse dieser Bearbeitung (also die Abstände bzw. Winkel zusammen mit den Koordinaten der Referenzpunkte und/oder die Ergebniswerte) wieder durch ein RIS oder PACS an den Anwender zurückgesandt.

Im Rahmen einer bevorzugten Ausführungsform der erfindungsgemäßen Vorrichtung sind die vorgenannten Einheiten (Datenschnittstelle, Referenzpunkt-Bestimmungseinheit und insbesondere auch die Ergebniswert-Ermittlungseinheit) auf Seiten der "Cloud" vorhanden. Ein bevorzugtes Ermittlungssystem umfasst zusätzlich zu einer solchen Vorrichtung noch eine lokale Recheneinheit, die über einen Datenkanal (z.B. ein Netzwerk, insbesondere als RIS oder PACS ausgestaltet) mit der Vorrichtung verbunden ist, wobei die lokale Rechnereinheit zumindest eine Datenempfangsschnittstelle aufweist, um Daten (insbesondere zu Abständen bzw. Winkeln zusammen mit den Koordinaten von Referenzpunkten und/oder die Ergebniswerte) zu empfangen. Zudem ist es bevorzugt, wenn der lokale Rechner zudem eine Sendeschnittstelle aufweist, um Daten an die Vorrichtung zu senden. Diese Sendeschnittstelle ist aber nicht unbedingt notwendig, wenn eine Architektur des Datenkanals vorliegt, die es erlaubt, eine Aufnahme eines medizintechnischen bildgebenden Systems (z.B. eines Röntgengerätes) zusammen mit einer Information zu der Art der gewünschten Ergebniswerten direkt der Vorrichtung zur Verfügung zu stellen.

Es kann im Rahmen der vorgenannten Ausführungsformen vorteilhaft sein, dass die bereitgestellte Rechenleistung von der Identität bzw. Auswahl des Anwenders abhängt. Beispielsweise können die zur Bestimmung der Referenzpunkte und/oder Abstände/Winkel einsetzbaren Algorithmen unterschiedliche Komplexitäten aufweisen und unterschiedlich genaue Ergebnisse hervorbringen. Der Anwender kann dabei bevorzugt den Grad der Bearbeitung durch die "Cloud" auswählen, insbesondere welcher Algorithmus verwendet werden soll bzw. welche Qualität das Ergebnis haben soll. Beispielsweise ist bei einer groben Übersicht die Anforderung an die Qualität vergleichsweise gering, so dass ein "schneller" Algorithmus ausgewählt werden kann, um möglichst schnell ein Ergebnis zu erhalten.

Wie oben bereits angesprochen wurde, kann zur automatischen Bestimmung der Referenzpunkte ein im Rahmen des maschinellen Lernens trainierter Algorithmus verwendet werden, bevorzugt ein Neuronales Netzwerk, insbesondere ausgelegt zum Deep (Machine) Learning. Die automatische Bestimmung von Referenzpunkten in der Bildaufnahme erfolgt dann mittels dieses Algorithmus. Der Algorithmus wird dabei vor der Ermittlung von Ergebniswerten auf Basis einer Vielzahl von skelettalen medizintechnischen Bildaufnahmen trainiert.

Zum Training wird vorteilhafter Weise eine große Anzahl von Bildaufnahmen mit bereits eingetragenen Referenzpunkten verwendet, z.B. Röntgenbilder mit von Radiologen bestimmten Referenzpunkten. Die Referenzpunkte sollten dabei möglichst ackurat eingetragen worden sein, damit das Training zeitlich optimiert vonstattengehen kann. Ein dermaßen trainierter Algorithmus kann dann automatisch Referenzpunkte auf neuen skelettalen Bildaufnahmen generieren.

Gemäß einem Aspekt der Erfindung kann in diesem Rahmen ein Trainings-Verfahren zum Erstellen einer Referenzpunkt-Bestimmungseinheit für eine erfindungsgemäße Vorrichtung bevorzugt sein, umfassend die Schritte:

- Bereitstellung einer Lern-Rechenvorrichtung, wobei die Lern-Rechenvorrichtung dazu ausgestaltet ist, einen im Rahmen des maschinellen Lernens trainierbaren Algorithmus, bevorzugt ein Neuronales Netzwerk, anhand von Bildaufnahmen mit eingetragenen Referenzpunkten zu trainieren,

- Bereitstellung einer Referenzpunkt-Bestimmungseinheit umfassend einen solchen Algorithmus, der jedoch untrainiert oder zumindest nicht optimal trainiert ist, auf oder an der Lern-Rechenvorrichtung.

- Bereitstellung einer Vielzahl von Bildaufnahmen mit eingetragenen Referenzpunkten,

- Trainieren des Algorithmus der Referenzpunkt-Bestimmungseinheit gemäß einem Prinzip des maschinellen Lernens, insbesondere basierend auf der Erkennung der Referenzpunkte in den Bildaufnahmen und Zuordnung von Mustern in den Bildaufnahmen zu den Positionen der Referenzpunkte.

Bevorzugt ist in dieser Hinsicht auch eine Lern-Rechenvorrichtung umfassend einen Prozessor und einen Datenspeicher mit Instruktionen, welche dem Prozessor bei ihrer Ausführung ermöglichen:

- Der Rechenvorrichtung bereitgestellte skelettale medizintechnische Bildaufnahmen mit eingetragenen Referenzpunkten zu erfassen.

- Die Referenzpunkte in den Bildaufnahmen als Objekte zu erkennen.

- Einen vorgenannten Algorithmus gemäß einem Prinzip des maschinellen Lernens zu trainieren, bevorzugt basierend auf der Erkennung der Referenzpunkte in den Bildaufnahmen und Zuordnung von Mustern in den Bildaufnahmen zu den Positionen der Referenzpunkte.

Ein erfindungsgemäßes Verfahren umfasst zusätzlich die folgenden Schritte:

- Anzeige der Bildaufnahme zusammen mit Referenzpunkten, und bevorzugt auch von Abständen und/oder Winkeln. Diese Darstellung erfolgt im Rahmen eines Kontrollbilds. Das Kontrollbild umfasst also die Bildaufnahme und die zusätzlich dargestellten Komponenten in Form von Bildinformationen.

- (Bevorzugt:) Bereitstellung einer manuellen Verschiebungsmöglichkeit für die Referenzpunkte in dem Kontrollbild. Unter der Verschiebungsmöglichkeit ist dabei eine Funktion (bzw. eine Einheit) zu verstehen, die es einem Benutzer ermöglicht, Referenzpunkte in dem Kontrollbild zu verschieben. Dazu umfasst die Verschiebungsmöglichkeit bevorzugt eine Abfrage eines Verschiebebefehls und/oder eines Bestätigungsbefehls, der eine Verschiebung bzw. Platzierung eines Referenzpunkts bewirkt.

Eine erfindungsgemäße Vorrichtung umfasst dafür eine Ausgebedatenschnittstelle zur Ausgabe von Bilddaten eines Kontrollbilds an eine Bildanzeigeeinheit.

Eine besonders bevorzugte Vorrichtung umfasst des Weiteren eine Verschiebungseinheit, welche für eine manuelle Verschiebungsmöglichkeit für die Referenzpunkte in dem Kontrollbild ausgelegt ist. Hierunter ist insbesondere eine Einheit zu verstehen, die dazu ausgelegt ist, eine Abfrage eines Verschiebebefehls und eines Bestätigungsbefehls zu verarbeiten und in Abhängigkeit von diesen Befehlen einen Referenzpunkt in dem Kontrollbild zu positionieren.

Erfindungsgemäß wird für einen, insbesondere für jeden, Referenzpunkt eine Referenzwertabhängigkeit RWA bestimmt. Diese Referenzwertabhängigkeit RWA repräsentiert dabei die Abhängigkeit eines orthopädischen Ergebniswerts von einer räumlichen Verschiebung des Referenzpunkts. Beispielsweise gibt die Referenzwertabhängigkeit RWA an, um wieviel der Ergebniswert abweichen würde, wenn man einen Referenzpunkt um einen (kleinen) Verschiebungsvektor $\hat{s}$ verschieben würde. Damit kann abgeschätzt werden, wie akkurat der Ergebniswert berechnet worden ist, selbst wenn die Referenzpunkte nicht optimal positioniert worden wären.

Die bestimmte Referenzwertabhängigkeit RWA wird bevorzugt zusammen mit dem Referenzpunkt (bzw. den betreffenden Referenzpunkten) visualisiert, insbesondere als eine Komponenten in dem vorgenannten Kontrollbild. Die Referenzwertabhängigkeit kann dabei als konkrete Werte dargestellt werden, die auch als "Referenzwertabhängigkeitswerte" bezeichnet werden können, als Schar von Punkten, die in einer Punktewolke entsprechend des betreffenden Verschiebungsvektors um einen Referenzpunkt herum angeordnet sind, und die jeweilige als Referenzwertabhängigkeit an diesem Punkt wiedergeben. Die Referenzwertabhängigkeit kann aber auch in Form von anderen grafischen Elementen visualisiert werden, wie z.B. Linien, Farbmarkierungen oder Schattierungen. Besonders bevorzugt bietet das Verfahren in Ergänzung die vorgenannte Verschiebungsmöglichkeit.

Eine bevorzugte Vorrichtung umfasst dafür eine Referenzwertabhängigkeits-Bestimmungseinheit zur Bestimmung einer Referenzwertabhängigkeit RWA für einen Referenzpunkt.

Bezüglich der Bestimmung der Referenzwertabhängigkeit RWA für einen Referenzpunkt werden bevorzugt die folgenden Schritte durchlaufen:

- Ein Basis-Ergebniswert wird aus einer Gruppe von für den betreffenden orthopädischen Ergebniswert relevanten Referenzpunkten gebildet.

- Ein Vergleichs-Ergebniswert wird aus der Gruppe von für den betreffenden orthopädischen Ergebniswert relevanten Referenzpunkten gebildet, wobei der Referenzpunkt, für den die Referenzwertabhängigkeit RWA bestimmt wird, zur Berechnung des Vergleichs-Ergebniswerts (um den vorgenannten Verschiebungsvektor $\hat{s}$) verschoben ist.

Dies kann für alle relevanten Referenzpunkte durchgeführt werden.

Bevorzugt wird eine absolute Referenzwertabhängigkeit aRWA bestimmt. Diese wird bevorzugt aus der Differenz aus Vergleichs-Ergebniswert und Basis-Ergebniswert bestimmt.

Besonders bevorzugt wird aus der absoluten Referenzwertabhängigkeit aRWA eine relative Referenzwertabhängigkeit rRWA bestimmt. Dies geschieht insbesondere dadurch, dass die absolute Referenzwertabhängigkeit aRWA mittels des Basis-Ergebniswerts normiert wird.

Der Basis-Ergebniswert und der Vergleichs-Ergebniswert werden bevorzugt nach der gleichen Weise berechnet wie der orthopädische Ergebniswert.

Die Referenzwertabhängigkeit RWA wird beispielsweise basierend auf einem Ergebniswert $M = M(L_1, L_2, ..., L_N)$ für einen Anzahl von N Referenzpunkten mit den Koordinaten $(L_1, L_2, ..., L_N)$ berechnet. Was den Ergebniswert M betrifft, existiert eine Vielzahl an unterschiedliche Messungen, die auf Röntgenbildern vorgenommen werden können. Die Art der Messung hängt z.B. von der diagnostischen Fragestellung bzw. des Körperbereiches ab. Die Funktion ist eine mathematische Beschreibung der Messung. Für eine gängige orthopädische Messung zur Bestimmung des Insall-Salvati-Index würde z.B. gelten: $M(L_1, L_2, L_3) = |L_1-L_2|/|L_2-L_3|$.

Eine absolute Referenzwertabhängigkeit aRWA für den k-ten Referenzpunkt $R_k$ mit dem Verschiebungsvektor $\hat{s}$ ergibt sich dann beispielsweise nach

$$aRWA(k,\hat{s}) = M(\acute{L}_1, \acute{L}_2, ..., \acute{L}_N) - M(L_1, L_2, ..., L_N), \qquad (1)$$

mit $\acute{L}_i = L_i$ wenn $i \neq k$ und $\acute{L}_i = L_i + \hat{s}$ wenn $i = k$.

Die relative Referenzwertabhängigkeit rRWA für den k-ten Referenzpunkt $R_k$ berechnet sich dann aus der absoluten Referenzwertabhängigkeit aRWA beispielsweise nach

$$rRWA(k,\hat{s}) = aRWA(k,\hat{s}) / M(L_1, L_2, ..., L_N). \qquad (2)$$

Bevorzugt werden für eine (relative) Referenzwertabhängigkeit RWA für einen Referenzpunkt, Isolinien ermittelt (und ggf. auch visualisiert). Diese Isolinien kennzeichnen die Positionen von Referenzpunkten, die einer bestimmten, möglicherweise absoluten, vorzugsweise jedoch prozentualen, Änderung des orthopädischen Ergebniswerts entsprechen. Betrachtet man das vorangehende Beispiel zur Berechnung der (relativen) Referenzwertabhängigkeit RWA, so können zur Ermittlung der Isolinien verschiedene Referenzwertabhängigkeiten mit unterschiedlichen Verschiebungsvektoren berechnet werden. Es können aber auch die Formeln umgestellt werden und für eine feste (ggf. prozentuale) Änderung die Menge der Verschiebungsvektoren bestimmt werden, die zu einer solchen (ggf. prozentualen) Änderung führen würden. Zu den Isolinien wird auch auf die untenstehenden Beispiele verwiesen.

Diese Isolinien werden bevorzugt in einem Kontrollbild visualisiert, bzw. angezeigt (z.B. als Werte, die auch als "Referenzwertabhängigkeitswerte" bezeichnet werden können oder in Form von grafischen Elementen) und dienen auf diese Weise als Hinweis darauf, welchen Einfluss Verschiebungen von Referenzpunkten haben könnten.

Bevorzugt wird für eine (relative) Referenzwertabhängigkeit RWA für einen Referenzpunkt, ein lokaler Akzeptanzbereich ermittelt (und ggf. auch visualisiert). Dieser lokale Akzeptanzbereich kennzeichnet denjenigen Bereich, innerhalb dessen eine Verschiebung des Referenzpunkts eine relative Änderung des orthopädischen Ergebniswerts innerhalb eines vorbestimmten globalen Akzeptanzbereichs hervorruft. Zu dem lokalen Akzeptanzbereich wird auch auf die untenstehenden Beispiele verwiesen.

Dieser lokale Akzeptanzbereich wird bevorzugt in einem Kontrollbild visualisiert, bzw. angezeigt (z.B. als Werte, die auch als "Referenzwertabhängigkeitswerte" bezeichnet werden können oder in Form von grafischen Elementen), insbesondere mit einer (farblichen) Kennzeichnung und dient auf diese Weise als Hinweis darauf, welche Verschiebungen von Referenzpunkten akzeptabel wären.

Bevorzugt wird zusätzlich eine automatische Klassifikation zumindest von Teilen der skelettalen medizintechnischen Bildaufnahme basierend auf den orthopädischen Ergebniswerten mittels Normbereichen vorgenommen. Diese Klassifikation klassifiziert den betreffenden Bildteil nach medizinischen Gesichtspunkten, wobei z.B. eine Fehlstellung, eine Vergrößerung oder eine Verkleinerung eines Körperbereichs festgestellt werden kann.

Die Klassifikation erfolgt bevorzugt anhand von Grenzwerten, welche die Normbereiche festlegen. Die Grenzwerte liegen dabei besonders bevorzugt in einer Datenbank vor. Liegt Beispielsweise der Fall vor, dass ein Ergebniswert ermittelt worden ist, der unterhalb eines Grenzwerts G liegt, so wäre der Ergebniswert unauffällig. Wenn der Ergebniswert jedoch gleich G ist oder diesen Wert übersteigt, dann könnte eine bestimmte Pathologie vorliegen, die mit dem gewählten Grenzwert auch medizinisch klar definiert werden könne. Die Normbereiche bzw. Grenzwerte können patientenabhängig sein. Beispielsweise können Normbereiche bzw. Grenzwerte altersabhängig, gewichtsabhängig, größenabhängig oder geschlechtsabhängig sein.

Bevorzugt werden für eine (relative) Referenzwertabhängigkeit RWA für einen Referenzpunkt, Klassengrenzen ermittelt (und ggf. auch visualisiert). Diese Klassengrenzen kennzeichnen die Bereiche innerhalb derer Verschiebungen des betreffenden Referenzpunkts lediglich (insbesondere relative) Änderungen des orthopädischen Ergebniswerts bewirken, die innerhalb einer Klassifikation liegen. Zu den Klassengrenzen wird auch auf die untenstehenden Beispiele verwiesen.

Diese Klassengrenzen werden bevorzugt in einem Kontrollbild visualisiert, bzw. angezeigt (z.B. als Werte, die auch als "Referenzwertabhängigkeitswerte" bezeichnet werden können oder in Form von grafischen Elementen) und dienen auf diese Weise als Hinweis darauf, welchen Einfluss Verschiebungen von Referenzpunkten haben könnten. Beispielsweise könnte bei einem nicht optimal positionierten Referenzpunkt auf einen Blick erkennbar sein, ob eine Nachjustierung der Position erfolgen sollte, oder ob der Aufwand dafür unnötig wäre, da sich an der Klassifizierung dadurch nichts ändern würde.

Eine diesbezüglich bevorzugte Vorrichtung umfasst eine Visualisierungseinheit zur Visualisierung von Isolinien und/oder von einem lokalen Akzeptanzbereich und/oder von Klassengrenzen. Diese Visualisierungseinheit kann als eine Steuereinrichtung zur Ansteuerung einer Anzeigeeinheit, z.B. eines Monitors ausgestaltet sein. Diese Steuereinrichtung ist dabei für eine Steuerung der Anzeigeeinheit ausgelegt demgemäß, dass Isolinien und/oder ein lokaler Akzeptanzbereich und/oder Klassengrenzen angezeigt werden.

Bevorzugt ist eine Kombination aus der oben beschriebenen Verschiebungsmöglichkeit mit einer Anzeige der Isolinien und/oder des lokalen Akzeptanzbereichs und/oder der Klassengrenzen, wobei besonders bevorzugt mit jedem Verschiebungsschritt die Isolinien und/oder der lokale Akzeptanzbereich und/oder die Klassengrenzen neu berechnet werden, da diese sich durch die Verschiebung eines Referenzpunktes auch für andere Referenzpunkte ändern könnten.

Gemäß eines bevorzugten Verfahrens erfolgt eine automatische Erstellung eines Berichts. Dieser Bericht enthält Informationen der Gruppe

- Orthopädische Ergebniswerte,
- Abstände,
- Winkel,
- Koordinaten der Referenzpunkte,
- eine Klassifikation, und bevorzugt
- weitere patientenspezifische Informationen.

Dabei wird bevorzugt eine computerlesbare Datei erzeugt, z.B. eine pdf-Datei, welche die Informationen in Tabellenform und/oder die Referenzpunkte bzw. Abstände und/oder Winkel als Abbildungen enthält. Eine bevorzugte Vorrichtung umfasst diesbezüglich eine Berichterstellungseinheit und insbesondere eine Software-Bibliothek, wobei die Berichterstellungseinheit dazu ausgelegt ist Datei mit dem betreffenden Bericht zu generieren.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1    eine Blockdarstellung einer bevorzugten Ausführungsform des erfindungsgemäßen Verfahrens,

Figur 2    eine Darstellung einer bevorzugten Vorrichtung,

Figur 3    ein Beispiel für ein von der Erfindung erstelltes Kontrollbild,

Figur 4    ein weiteres Beispiel für ein von der Erfindung erstelltes Kontrollbild umfassend Isolinien,

Figur 5    ein weiteres Beispiel für ein von der Erfindung erstelltes Kontrollbild umfassend lokale Akzeptanzbereiche,

Figur 6    ein weiteres Beispiel für ein von der Erfindung erstelltes Kontrollbild umfassend Klassengrenzen,

Figur 7    eine grob schematische Darstellung eines bildgebenden Systems mit einem Ausführungsbeispiel einer erfindungsgemäßen Steuereinrichtung zur Durchführung des Verfahrens.

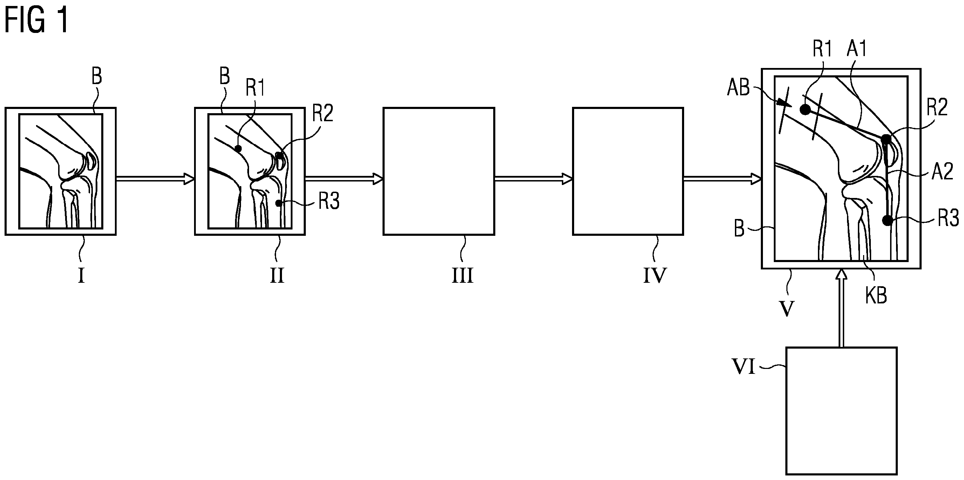

Figur 1 zeigt eine Blockdarstellung einer bevorzugten Ausführungsform des erfindungsgemäßen Verfahrens zur Ermittlung von Ergebniswerten auf Basis einer skelettalen medizintechnischen Bildaufnahme B.

In Schritt I erfolgt eine Bereitstellung einer skelettalen medizintechnischen Bildaufnahme B, wobei hier die Aufnahme eines menschlichen Knies gezeigt ist.

In Schritt II erfolgt eine automatische Bestimmung von Referenzpunkten R1, R2, R3 in der Bildaufnahme B, die hier als Kreise dargestellt sind.

In Schritt III erfolgt eine Berechnung eines orthopädischen Ergebniswerts OE basierend zumindest auf einem Abstand A1, A2 zwischen zwei Referenzpunkten R1, R2, R3 und/oder auf einem von Referenzpunkten R1, R2, R3 definierten Winkel.

In Schritt IV erfolgt eine Bestimmung von jeweils einer Referenzwertabhängigkeit RWA für die in Schritt II bestimmten Referenzpunkte R1, R2, R3.

Diese Referenzwertabhängigkeit RWA wird für einen der Referenzpunkte R1, R2, R3 so bestimmt, dass basierend auf der Berechnung des orthopädischen Ergebniswerts OE ein Basis-Ergebniswert berechnet wird und ein Vergleichs-Ergebniswert berechnet wird, wobei zur Berechnung des Vergleichs-Ergebniswerts der betrachtete Referenzpunkt R1, R2, R2 um einen Verschiebungsvektor verschoben wird. Aus der Differenz aus Basis-Ergebniswert und Vergleichs-Ergebniswert normiert auf den Basis-Ergebniswert ergibt sich dann die Referenzwertabhängigkeit RWA, die in diesem Fall eine relative Referenzwertabhängigkeit rRWA ist.

In Schritt V erfolgt eine Anzeige eines Kontrollbildes KB, welches die Bildaufnahme B zusammen mit Referenzpunkten R1, R2, R3 und Abständen A1, A2 und zusätzlich einem aus der Referenzwertabhängigkeit RWA abgeleiteten Akzeptanzbereich AB umfasst.

In Schritt VI wird eine manuellen Verschiebungsmöglichkeit für die Referenzpunkte R1, R2, R3 in dem Kontrollbild KB zur Verfügung gestellt, der einen Benutzer die Referenzpunkte R1, R2, R3 nach Belieben verschieben lässt. Bevorzugt erfolgt bei einer Verschiebung eine erneute Bestimmung und Visualisierung der relativen Referenzwertabhängigkeit rRWA in dem Kontrollbild basierend auf den verschobenen Referenzpunkten R1, R2, R3.

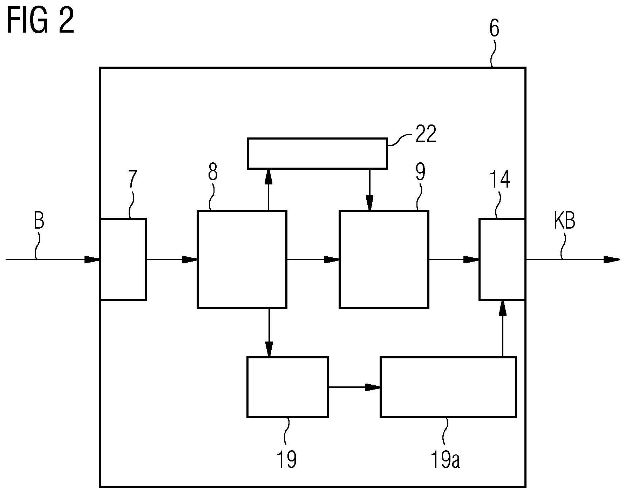

Figur 2 skizziert eine bevorzugte Vorrichtung 6 zur Ermittlung von Ergebniswerten OE auf Basis einer skelettalen medizintechnischen Bildaufnahme B. Diese Vorrichtung 6 umfasst die folgenden Komponenten:

Eine Datenschnittstelle 7 zur Erfassung von Bilddaten einer skelettalen medizintechnischen Bildaufnahme B, z.B. über ein Radiologieinformationssystem ("RIS") aus einem PACS (Bildarchivierungs- und Kommunikationssystem) oder einer medizinischen Bildaufnahmeeinrichtung. Da die Daten in diesem Beispiel digitaler Natur sind, kann hier die Bildaufnahme B mit den Bilddaten gleichgesetzt werden.

Eine Referenzpunkt-Bestimmungseinheit 8 ausgelegt für eine automatische Bestimmung von Referenzpunkten R1, R2, R3 in der Bildaufnahme B.

Eine Ergebniswert-Ermittlungseinheit 9 ausgelegt für eine Berechnung eines orthopädischen Ergebniswerts OE basierend zumindest auf einem Abstand A1, A2 zwischen zwei Referenzpunkten R1, R2, R3 und/oder auf einem von Referenzpunkten R1, R2, R3 definierten Winkel.

Zusätzlich umfasst die Vorrichtung 6 noch die folgenden Komponenten:

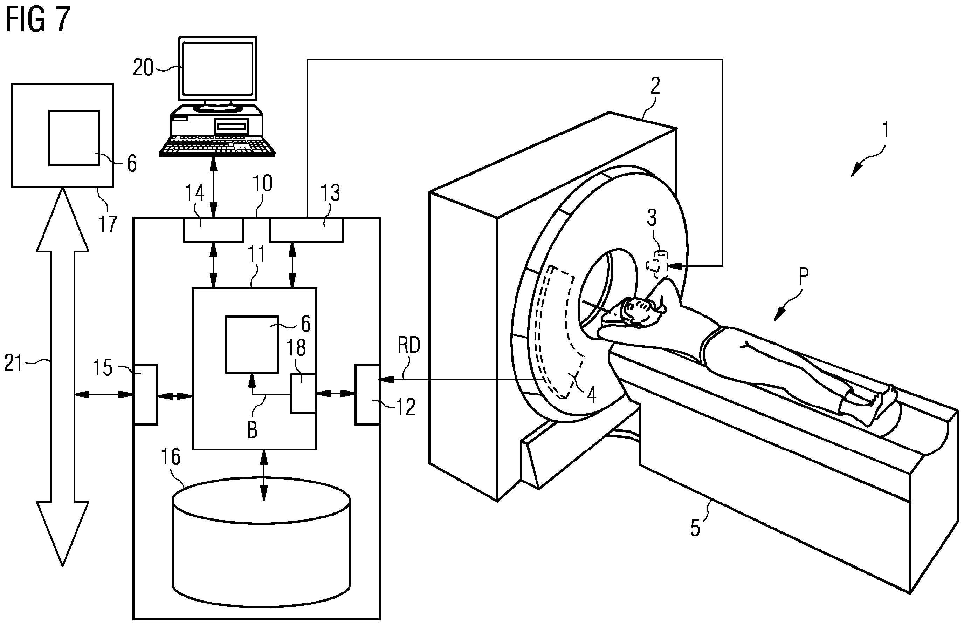

Eine Ausgebedatenschnittstelle 14 zur Ausgabe von Bilddaten eines Kontrollbilds KB an eine Bildanzeigeeinheit 20, z.B. ein Computerterminal 20, wie es in Figur 7 dargestellt ist.

Eine Referenzwertabhängigkeits-Bestimmungseinheit 19 zur Bestimmung einer Referenzwertabhängigkeit RWA für die Referenzpunkte R1, R2, R3 und optional zusätzlich eine Visualisierungseinheit 19a zur Visualisierung von Isolinien I, einem lokalen Akzeptanzbereich A bzw. von Klassengrenzen KG (siehe nachfolgende Figuren). Diese Visualisierungseinheit 19a ist als Steuereinrichtung zur Ansteuerung einer Anzeigeeinheit 20 ausgestaltet. Diese steuert die Anzeigeeinheit so, dass Isolinien I, lokale Akzeptanzbereiche A bzw. Klassengrenzen KG angezeigt werden.

In dem hier dargestellten Beispiel erhält die Referenzwertabhängigkeits-Bestimmungseinheit 19 ihre Daten von der Referenzpunkt-Bestimmungseinheit 8, bestimmt die Referenzwertabhängigkeit RWA und gibt die zu visualisierenden Daten an die Ausgebedatenschnittstelle 14 weiter. Es sind auch Ausführungsformen denkbar, bei denen die Referenzwertabhängigkeits-Bestimmungseinheit 19, insbesondere zusammen mit der Visualisierungseinheit 19a Teil der Ergebniswert-Ermittlungseinheit 9 sind.

Eine optionale Verschiebungseinheit 21, die für eine manuelle Verschiebungsmöglichkeit für die Referenzpunkte R1, R2, R3 ausgelegt ist. Sie erhält in diesem Beispiel ihre Daten von der Referenzpunkt-Bestimmungseinheit 8 und liefert Daten zu den verschobenen Referenzpunkten R1, R2, R3 an die Ergebniswert-Ermittlungseinheit 9, so dass neue Ergebniswerte basierend auf den verschobenen Referenzpunkten berechnet werden können. In anderen Beispielen kann die Ausgabe der Verschiebungseinheit 21 auch mit der Referenzwertabhängigkeits-Bestimmungseinheit 19 gekoppelt sein, so dass basierend auf den verschobenen Referenzpunkten auch angepasste Referenzwertabhängigkeiten RWA berechnet werden können. Die Verschiebungseinheit 21 kann auch Teil der Ergebniswert-Ermittlungseinheit 9 oder der Referenzwertabhängigkeits-Bestimmungseinheit 19 sein.

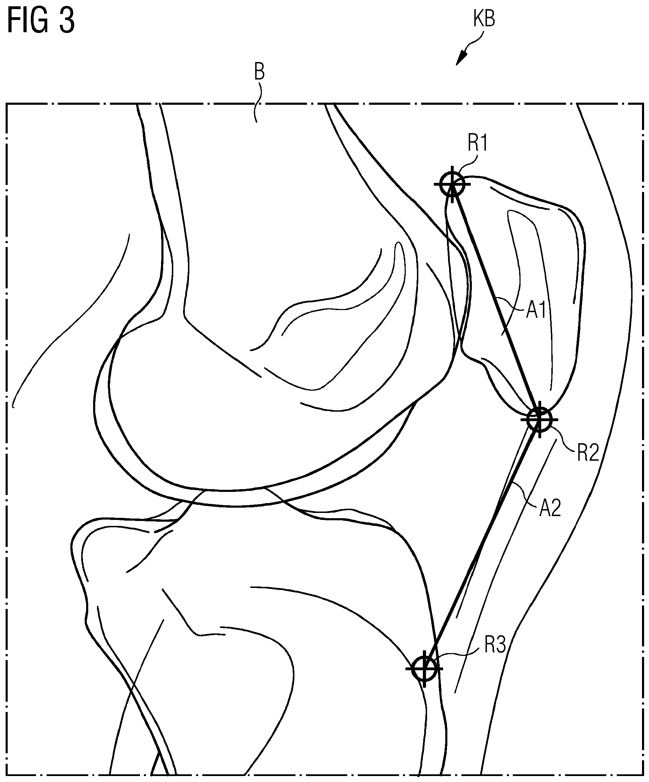

Figur 3 zeigt ein Beispiel für ein von der Erfindung erstelltes Kontrollbild KB aus einer skelettalen Bildaufnahme B eines menschlichen Kniegelenks. In der Bildaufnahme B wurden die Punkte "Basis Patellae", "Apex Patellae" und "Tuberositas Tibiae" als Referenzpunkte R1, R2, R3 markiert. Aus den in der Figur eingezeichneten Abständen A1, A2 der Referenzpunkte R1, R2, R3 lässt sich als orthopädischer Ergebniswert OE beispielsweise der Insall-Salvati-Index aus dem im Bild unten eingezeichneten Abstand A2 zwischen "Apex Patellae" und "Tuberositas Tibiae" dividiert durch den im Bild oberen Abstand zwischen "Basis Patellae" und "Apex Patellae". Der Insall-Salvati-Index liegt bei einem gesunden Knie zwischen 0,8 und 1,2. Wenn ein Insall-Salvati-Index unter 0,8 festgestellt wird, kann auf eine Patellasehnenruptur geschlossen werden, liegt er über 1,2 könnte eine Quadrizepssehnenruptur vorliegen.

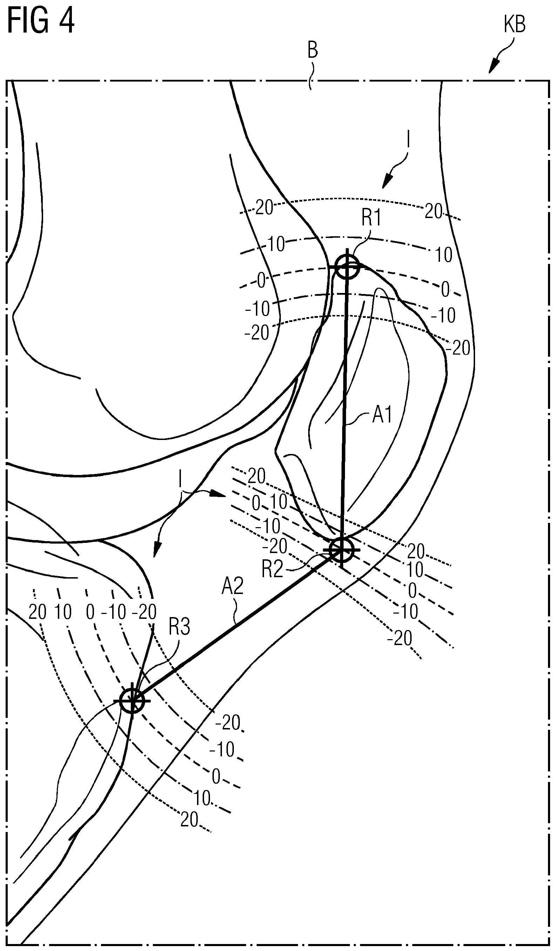

In den nachfolgenden Beispielen, die durch die Figuren 4, 5 und 6 verdeutlicht werden. Liegt genau wie in Figur 3 eine Bildaufnahme B des menschlichen Knies vor, in welcher die Punkte "Basis Patellae", "Apex Patellae" und "Tuberositas Tibiae" als Referenzpunkte R1, R2, R3 markiert und deren Abstände A1, A2 eingezeichnet wurden. Als orthopädischer Ergebniswert OE wird wieder der Insall-Salvati-Index betrachtet.

Für die einzelnen Referenzpunkte R1, R2, R3 kann nun ein jeweilige Referenzwertabhängigkeit RWA ermittelt werden. Zur Berechnung einer absoluten Referenzwertabhängigkeit aRWA wird z.B. zunächst als Basis-Ergebniswert der Insall-Salvati-Index, also der unten eingezeichneten Abstand A2 zwischen "Apex Patellae" und "Tuberositas Tibiae" dividiert durch den oberen Abstand zwischen "Basis Patellae" und "Apex Patellae" für die Referenzpunkte normal berechnet, anschließend wird als ein Vergleichs-Ergebniswerte der Insall-Salvati-Index berechnet während einer der Referenzpunkte R1, R2, R3 jeweils um einen Verschiebungsvektor $\hat{s}$ verschoben wird. Im Anschluss an diese Berechnungen wird dann nach der obigen Formel (1) die absolute Referenzwertabhängigkeit aRWA für einen Referenzpunkt R1, R2, R3 mit

$$aRWA = \text{Vergleichs-Ergebniswert} - \text{Basis-Ergebniswert}$$

berechnet. Eine relative Referenzwertabhängigkeit rRWA kann nach obiger Formel (2) aus

$$rRWA = aRWA\ /\ Basis\text{-}Ergebniswert$$

also

$$rRWA = (Vergleichs\text{-}Ergebniswert\ /\ Basis\text{-}Ergebniswert)\ -\ 1$$

ermittelt werden.

Werden diese Berechnungen nun für alle Referenzpunkte R1, R2, R3 mit einer Vielzahl von Verschiebungsvektoren $\hat{s}$ durchgeführt, so erhält man in einem Bereich um die Referenzpunkte R1, R2, R3 herum (der durch die Verschiebungsvektoren $\hat{s}$ vorgegeben wird) viele mögliche Referenzwertabhängigkeiten RWA.

Figur 4 zeigt ein Beispiel für ein von der Erfindung erstellten Kontrollbild KB umfassend Isolinien I. Diese Isolinien I geben mögliche verschobene Positionen der Referenzpunkte R1, R2, R3 zusammen mit den relativen Referenzwertabhängigkeiten rRWA an diesen Positionen an. Konkret verläuft eine Isolinie I entlang derjenigen Positionen, an denen die relative Referenzwertabhängigkeit rRWA jeweils gleich ist.

Die besondere Visualisierung in Figur 4 mittels Isolinien I zeigt also, in welcher Richtung eine Verschiebung eines Referenzpunkts R1, R2, R3 (einer Landmarke) große Änderungen oder kleine Änderungen am Ergebniswert OE erzeugt. In der Richtung entlang einer Isolinie findet keine Veränderung statt, in einer Richtung orthogonal zu den Isolinien findet eine große Veränderung statt. Zudem kann bei dieser Darstellung dem Kontrollbild KB sehr leicht entnommen werden, wie stark die Änderung bei einer Verschiebung entlang dieser Richtung ist. Ein großer Abstand zwischen Isolinien I steht für eine vergleichsweise geringe Änderung (bei einer Verschiebung orthogonal zu den Isolinien I), ein kleiner Abstand zwischen Isolinien I steht für eine relativ große Änderung. Insgesamt kann ein Arzt also besser die Richtungsabhängigkeit von Verschiebungen der Referenzpunkte R1, R2, R3 und die Auswirkung auf den Ergebniswert OE verstehen und dieses Verständnis zur Kontrolle der Landmarkpositionen (Position der Referenzpunkte R1, R2, R3) verwenden.

Ein großer Vorteil ist, dass der Arzt bei der Kontrolle der Referenzpunkte R1, R2, R3 eine höhere Aufmerksamkeit auf diejenigen Referenzpunkte R1, R2, R3 richten kann, bei denen eine Verschiebung eine höhere Änderung des Ergebniswerts OE hervorruft.

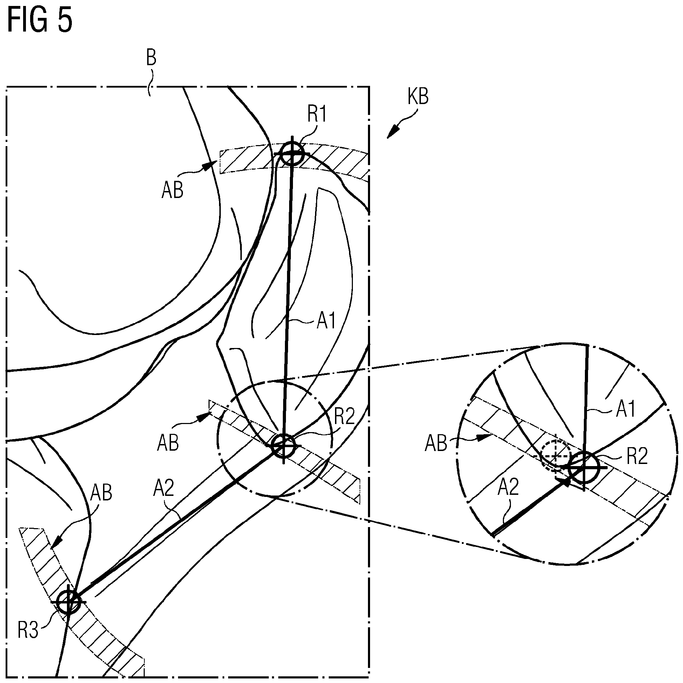

Figur 5 zeigt ein weiteres Beispiel für ein von der Erfindung erstellten Kontrollbild KB umfassend lokale Akzeptanzbereiche AB. Diese Visualisierung zeigt, ob eine Verschiebung eines Referenzpunkts R1, R2, R3 vernachlässigbare Veränderungen am Ergebniswert OE hervorruft. Zu beachten ist, dass gleichzeitige Verschiebungen von mehreren Referenzpunkten R1, R2, R3 zu einem Ergebniswert OE außerhalb des Akzeptanzbereiches führen können.

Ein Vorteil dieser Darstellung ist, dass in einem Fall, in dem ein Referenzpunkt R1, R2, R3 nicht optimal positioniert ist, die alternative Position aber innerhalb des lokalen Akzeptanzbereiches liegt, dies für den Arzt mit einem Blick auf das Kontrollbild offensichtlich ist. Wie in der Figur dargestellt, muss der in der Vergrößerung dargestellte Referenzpunkt R2 nicht unbedingt in Pfeilrichtung auf die korrekte Position (gestrichelt dargestellt) verschoben werden, da eine solche Verschiebung an dem Ergebniswert OE nichts ändern würde.

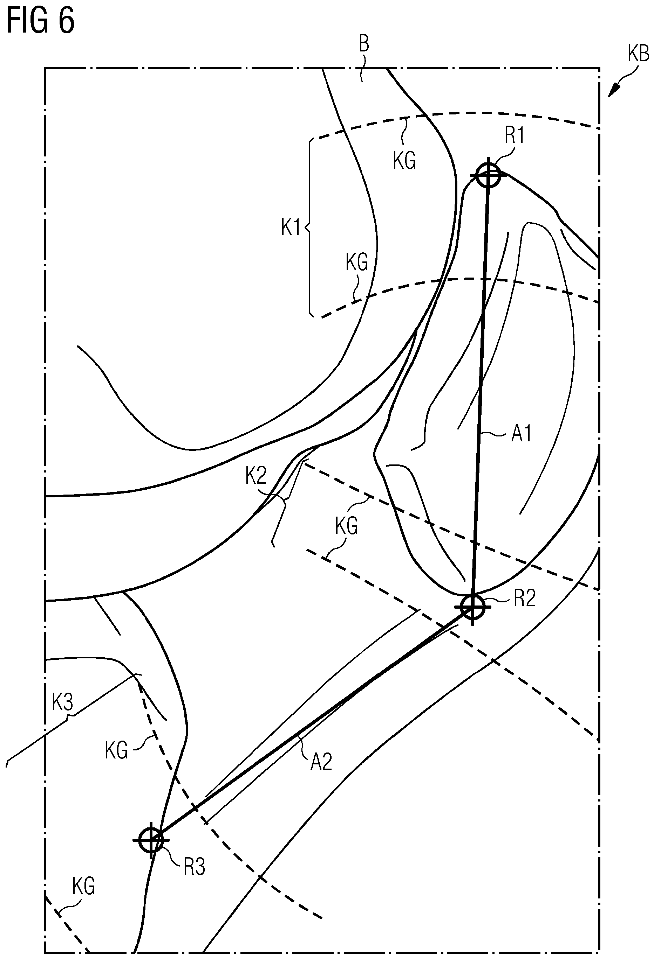

Figur 6 zeigt ein weiteres Beispiel für ein von der Erfindung erstellten Kontrollbild KB umfassend Klassengrenzen KG. Ein Ergebniswert OE kann generell in die Klassen normal und abnormal klassifiziert werden. In dem Beispiel ist für jeden Referenzpunkt R1, R2, R3 der Klassifizierungsbereich K1, K2, K3 dargestellt, in dem eine Position des Referenzpunkts R1, R2, R3 zu einem Ergebniswert OE führt, der noch im Normbereich liegt. Die Normbereiche müssen vorher bekannt sein. Zur (ggf. patientenabhängigen) Definition dieser Normbereiche existiert in der Regel Literatur.

Ein Vorteil dieser Darstellung ist, dass in einem Fall, dass ein Referenzpunkt R1, R2, R3 nicht optimal positioniert ist, die alternative Position aber keine Veränderung der Klassifizierung hervorruft, dies für den Arzt mit einem Blick auf das Kontrollbild offensichtlich ist. Somit kann eine Verschiebung des Referenzpunkt R1, R2, R3 unter Umständen unterlassen werden, da sie nicht notwendig ist.

Bei den folgenden Erläuterungen wird davon ausgegangen, dass es sich bei der bildgebenden Anlage um ein Computertomographie-System handelt. Grundsätzlich ist das Verfahren aber auch an anderen bildgebenden Anlagen einsetzbar. Die meisten orthopädischen Messungen werden in der Praxis auf 2D-Bildern durchgeführt. Dies können Schnittbilder eines CT sein oder Röntgenbilder, die mittels eines Radiographiesystems aufgenommen worden sind.

Figur 7 zeigt grob schematisch ein Computertomographiesystem 1 mit einer Steuereinrichtung 10 und einer Befundungsstation 17, wobei sowohl die Steuereinrichtung 10 als auch die Befundungsstation 17 eine erfindungsgemäße Vorrichtung 6 umfassen (zur besseren Übersicht ohne die in ihr enthaltenen Komponenten dargestellt) und somit zur Durchführung des erfindungsgemäßen Verfahrens geeignet sind. Das Computertomographiesystem 1 weist in üblicher Weise einen Scanner 2 mit einer Gantry auf, in der eine Röntgenquelle 3 rotiert, die jeweils einen Patienten durchstrahlt, welcher mittels einer Liege 5 in einen Messraum der Gantry hineingeschoben wird, so dass die Strahlung auf einen der Röntgenquelle 3 jeweils gegenüberliegenden Detektor 4 trifft. Es wird ausdrücklich darauf hingewiesen, dass es sich bei dem Ausführungsbeispiel gemäß Figur 7 nur um ein Beispiel eines CTs handelt und die Erfindung auch an beliebigen CT-Konstruktionen, beispielsweise mit ringförmigem feststehendem Röntgendetektor und/oder mehreren Röntgenquellen genutzt werden kann.

Ebenso sind bei der Steuereinrichtung 10 nur die Komponenten dargestellt, die für die Erläuterung der Erfindung hilfreich sind. Grundsätzlich sind derartige CT-Systeme und zugehörige Steuereinrichtungen dem Fachmann bekannt und brauchen daher nicht im Detail erläutert zu werden.

Eine Kernkomponente der Steuereinrichtung 10 ist hier ein Prozessor 11, auf dem verschiedene Komponenten in Form von Softwaremodulen realisiert sind. Die Steuereinrichtung 10 weist weiterhin eine Terminalschnittstelle 14 auf, an die ein Terminal 20 bzw. eine Anzeigeeinheit 20 angeschlossen ist, über das ein Bediener die Steuereinrichtung 10 und somit das Computertomographiesystem 1 bedienen kann und sich ggf. auch Bildaufnahmen ansehen kann. Eine weitere Schnittstelle 15 ist eine Netzwerkschnittstelle zum Anschluss an einen Datenbus 21, um so eine Verbindung zu einem RIS bzw. PACS und damit zur Befundungsstation 17 herzustellen. Über diesen Bus 21 können beispielsweise Bildaufnahmen B für die Befundungsstation 17 bereitgestellt werden.

Über eine Steuerschnittstelle 13 kann von der Steuereinrichtung 10 der Scanner 2 angesteuert werden. Über eine Akquisitionsschnittstelle 12 werden die Rohdaten RD aus dem Detektor 4 ausgelesen. Weiterhin weist die Steuereinrichtung 10 eine Speichereinheit 16 auf.

Als eine Softwarekomponente ist auf dem Prozessor 11 eine Bilddaten-Rekonstruktionseinheit 18 implementiert, mit welcher aus den über die Datenakquisitions-Schnittstelle 12 erhaltenen Rohdaten RD die gewünschten für die Bildaufnahme B rekonstruiert werden.

Das erfindungsgemäße Verfahren kann in diesem Beispiel direkt an dem Computertomographiesystem 1 ausgeführt werden, wobei Bildaufnahmen B angefertigt werden, diese direkt nach ihrer Rekonstruktion für das Verfahren bereitgestellt werden und die Kontrollbilder KB auf dem Computerterminal angezeigt werden. Es ist aber auch möglich, die Bildaufnahmen in Ruhe an der Befundungsstation 17 mit dem erfindungsgemäßen Verfahren zu bearbeiten.

Es wird abschließend noch einmal darauf hingewiesen, dass es sich bei den vorhergehend detailliert beschriebenen Verfahren sowie bei den dargestellten Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

**Patentansprüche**

1. Verfahren zur Ermittlung von Ergebniswerten auf Basis einer skelettalen medizintechnischen Bildaufnahme (B), **gekennzeichnet durch** die Schritte:

   - Bereitstellung einer skelettalen medizintechnischen Bildaufnahme (B),
   - automatische Bestimmung von Referenzpunkten (R1, R2, R3) in der Bildaufnahme (B),
   - Anzeige der Bildaufnahme (B) zusammen mit Referenzpunkten (R1, R2, R3) im Rahmen eines Kontrollbilds (KB), und
   - Bestimmung einer Referenzwertabhängigkeit für einen Referenzpunkt (R1, R2, R3), welche die Abhängigkeit eines orthopädischen Ergebniswerts (OE) von einer räumlichen Verschiebung des Referenzpunkts (R1, R2, R3) repräsentiert,
   - Berechnung eines orthopädischen Ergebniswerts (OE) basierend zumindest auf einem Abstand (A1, A2) zwischen zwei Referenzpunkten (R1, R2, R3) und/oder auf einem von Referenzpunkten (R1, R2, R3) definierten Winkel.

2. Verfahren nach Anspruch 1, umfassend die zusätzlichen Schritte:

   - Anzeige der Bildaufnahme (B) auch zusammen mit Abständen (A1, A2) und/oder Winkeln im Rahmen des Kontrollbilds (KB), und
   - Bereitstellung einer manuellen Verschiebungsmöglichkeit für die Referenzpunkte (R1, R2, R3) in dem Kontrollbild (KB).

3. Verfahren nach einem der vorangehenden Ansprüche, wobei die Referenzwertabhängigkeit zusammen mit dem Referenzpunkt (R1, R2, R3) visualisiert wird.

4. Verfahren nach Anspruch 3, wobei zur Bestimmung der Referenzwertabhängigkeit für einen Referenzpunkt (R1, R2, R3)

- ein Basis-Ergebniswert aus einer Gruppe von für den betreffenden orthopädischen Ergebniswert (OE) relevanten Referenzpunkten (R1, R2, R3) gebildet wird,
- ein Vergleichs-Ergebniswert aus der Gruppe von für den betreffenden orthopädischen Ergebniswert (OE) relevanten Referenzpunkten (R1, R2, R3) gebildet wird, wobei der Referenzpunkt (R1, R2, R3), für den die Referenzwertabhängigkeit bestimmt wird, zur Berechnung des Vergleichs-Ergebniswerts verschoben ist,

wobei bevorzugt eine absolute Referenzwertabhängigkeit aus der Differenz aus Vergleichs-Ergebniswert und Basis-Ergebniswert bestimmt wird und
besonders bevorzugt daraus eine relative Referenzwertabhängigkeit aus der absoluten Referenzwertabhängigkeit normiert durch den Basis-Ergebniswert bestimmt wird,
wobei der Basis-Ergebniswert und der Vergleichs-Ergebniswert bevorzugt nach der gleichen Weise wie der orthopädische Ergebniswert (OE) berechnet werden.

5. Verfahren nach Anspruch 3 oder 4, wobei für eine, insbesondere relative, Referenzwertabhängigkeit für einen Referenzpunkt (R1, R2, R3), Isolinien (I) ermittelt werden, welche die

Positionen kennzeichnen, die einer bestimmten, vorzugsweise prozentualen, Änderung des orthopädischen Ergebniswerts (OE) entsprechen,
und bevorzugt diese Isolinien (I) in einem Kontrollbild (KB) angezeigt werden.

6. Verfahren nach einem der Ansprüche 3 bis 5, wobei für eine, insbesondere relative, Referenzwertabhängigkeit für einen Referenzpunkt (R1, R2, R3) ein lokaler Akzeptanzbereich (AB) ermittelt wird, innerhalb dessen eine Verschiebung des Referenzpunkts (R1, R2, R3) eine relative Änderung des orthopädischen Ergebniswerts (OE) innerhalb eines vorbestimmten globalen Akzeptanzbereichs hervorruft,
und bevorzugt dieser lokale Akzeptanzbereich (AB) in einem Kontrollbild (KB) angezeigt wird.

7. Verfahren nach einem der vorangehenden Ansprüche, wobei zusätzlich eine automatische Klassifikation zumindest von Teilen der skelettalen medizintechnischen Bildaufnahme (B) basierend auf dem orthopädischen Ergebniswert (OE) und/oder der Koordinaten der Referenzpunkte (R1, R2, R3) mittels Normbereichen vorgenommen wird,

wobei die Klassifikation bevorzugt anhand von Grenzwerten erfolgt, welche die Normbereiche festlegen,
wobei die Grenzwerte besonders bevorzugt in einer Datenbank vorliegen.

8. Verfahren nach Anspruch 7, wobei für eine, insbesondere relative, Referenzwertabhängigkeit für einen Referenzpunkt (R1, R2, R3) Klassengrenzen (KG) ermittelt werden, innerhalb derer Verschiebungen des Referenzpunkts (R1, R2, R3) lediglich Änderungen des orthopädischen Ergebniswerts (OE) bewirken, die innerhalb einer Klassifikation liegen,
und bevorzugt diese Klassengrenzen (KG) in einem Kontrollbild (KB) angezeigt werden.

9. Verfahren nach Anspruch 7 oder 8, umfassend eine automatische Erstellung eines Berichts enthaltend Informationen der Gruppe

- Orthopädische Ergebniswerte (OE)
- Abstände (A1, A2),
- Winkel
- Koordinaten der Referenzpunkte (R1, R2, R3) und
- eine Klassifikation, und
- bevorzugt weitere patientenspezifische Informationen, wobei dazu bevorzugt eine computerlesbare Datei erzeugt wird, welche die Informationen in Tabellenform und/oder die Referenzpunkte (R1, R2, R3) als Abbildungen enthält.

10. Vorrichtung (6) zur Ermittlung von Ergebniswerten auf Basis einer skelettalen medizintechnischen Bildaufnahme (B), **dadurch gekennzeichnet, dass** die Vorrichtung

- eine Datenschnittstelle (7) zur Erfassung von Bilddaten einer skelettalen medizintechnischen Bildaufnahme (B),
- eine Referenzpunkt-Bestimmungseinheit (8) ausgelegt für eine automatische Bestimmung von Referenzpunkten (R1, R2, R3) in der Bildaufnahme (B),
- eine Ausgabedatenschnittstelle (14) zur Ausgabe von Bilddaten eines Kontrollbilds (KB) an eine Bildanzeigeeinheit (20),
- eine Referenzwertabhängigkeits-Bestimmungseinheit (19) zur Bestimmung einer Referenzwertabhängigkeit für einen Referenzpunkt (R1, R2, R3), welche die Abhängigkeit eines orthopädischen Ergebniswerts (OE) von einer räumlichen Verschiebung des Referenzpunkts (R1, R2, R3) repräsentiert,
- eine Ergebniswert-Ermittlungseinheit (9) ausgelegt für eine Berechnung eines orthopädischen Ergebniswerts (OE) basierend zumindest auf einem Abstand (A1, A2) zwischen zwei Referenzpunkten (R1, R2, R3) und/oder auf einem von Referenzpunkten (R1, R2, R3) definierten Winkel umfasst.

11. Vorrichtung (6) nach Anspruch 10, umfassend

- eine Verschiebungseinheit (22) ausgelegt für eine manuelle Verschiebungsmöglichkeit für die Referenzpunkte (R1, R2, R3) in einem Kontrollbild (KB),
- die Referenzwertabhängigkeits-Bestimmungseinheit (19) zur Bestimmung der Referenzwertabhängigkeit für den Referenzpunkt (R1, R2, R3) zusammen mit einer Visualisierungseinheit (19a) zur Visualisierung von Isolinien (I) und/oder einem lokaler Akzeptanzbereich (AB) und/oder Klassengrenzen (KG).

12. Befundungsstation (17) zur Befundung einer skelettalen medizintechnischen Bildaufnahme (B), welche zur Durchführung eines Verfahrens nach einem der Ansprüche 1 bis 9 ausgestaltet ist und/oder eine Vorrichtung nach Anspruch 10 oder 11 umfasst.

13. Medizintechnisches bildgebendes System (1) umfassend eine Vorrichtung nach Anspruch 10 oder 11 und/oder eine Befundungsstation (17) nach Anspruch 12.

14. Computerprogrammprodukt mit einem Computerprogramm, welches direkt in eine Speichereinrichtung eines Rechensystems oder einer Befundungsstation (17) eines medizintechnischen bildgebenden Systems (1) ladbar ist, mit Programmabschnitten, um alle Schritte des Verfahrens nach einem der Ansprüche 1 bis 9 auszuführen, wenn das Computerprogramm in dem Rechensystem oder der Befundungsstation (17) ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 9 auszuführen, wenn die Programmabschnitte von der Rechnereinheit ausgeführt werden.

**Claims**

1. Method for determining result values on the basis of a skeletal medical imaging recording (B), comprising the steps:

- providing a skeletal medical imaging recording (B),
- automatically determining reference points (R1, R2, R3) in the image recording (B),
- displaying the image recording (B) together with reference points (R1, R2, R3) within the context of a monitoring image (KB), and
- determining a reference value dependency for a reference point (R1, R2, R3), which represents the dependency of an orthopaedic result value (OE) on a spatial displacement of the reference point (R1, R2, R3),
- calculating an orthopaedic result value (OE) based at least upon a spacing (A1, A2) between two reference points (R1, R2, R3) and/or upon an angle defined by reference points (R1, R2, R3).

2. Method according to claim 1, comprising the additional steps:

- displaying the image recording (B) also together with spacings (A1, A2) and/or angles within the context of a monitoring image (KB), and
- providing a manual displacement capability for the reference points (R1, R2, R3) in the monitoring image (KB).

3. Method according to one of the preceding claims, wherein the reference value dependency preferably is visualised together with the reference point (R1, R2, R3).

4. Method according to claim 3, wherein for determining the reference value dependency for a reference point (R1, R2, R3)

- a basic result value is formed from a group of reference points (R1, R2, R3) relevant for the orthopaedic result value (OE) in question,
- a comparison result value is formed from the group of reference points (R1, R2, R3) of relevance for the orthopaedic result value (OE) in question, wherein the reference point (R1, R2, R3) for which the reference value dependency is determined is displaced for the calculation of the comparison result value,

wherein an absolute reference value dependency is determined from the difference of the comparison result value and the basic result value, and

particularly preferably, therefrom a relative reference value dependency is determined from the absolute reference value dependency, normalised by means of the basic result value, wherein the basic result value and the comparison result value are preferably calculated in the same way as the orthopaedic result value (OE).

5. Method according to claim 3 or 4, wherein for an, in particular relative, reference value dependency for a reference point (R1, R2, R3), isolines (I) are determined, which

identify the positions which correspond to a particular, preferably percentage, change of the orthopaedic result value (OE),
and preferably these isolines (I) are displayed in a monitoring image (KB).

6. Method according to one of the claims 3 to 5, wherein for an, in particular relative, reference value dependency for a reference point (R1, R2, R3) a local acceptance range (AB) is determined within which a displacement of the reference point (R1, R2, R3) brings about a relative change of the orthopaedic result value (OE) within a predetermined global acceptance range,
and preferably this local acceptance range (AB) is displayed in a monitoring image (KB).

7. Method according to one of the preceding claims, wherein an automatic classification at least of parts of the skeletal medical imaging recording (B) is additionally carried out on the basis of the orthopaedic result value (OE) and/or the coordinates of the reference points (R1, R2, R3) by means of normal ranges,

wherein the classification preferably takes place on the basis of limit values which define the normal ranges,
wherein the limit values particularly preferably are provided in a database.

8. Method according to claim 7, wherein for an, in particular relative, reference value dependency for a reference point (R1, R2, R3), class limits (KG) are determined, within which displacements of the reference point (R1, R2, R3) merely bring about changes in the orthopaedic result value (OE) which lie within a classification,
and preferably these class limits (KG) are displayed in a monitoring image (KB).

9. Method according to claim 7 or 8, comprising an automatic generation of a report comprising information items belonging to the group

- orthopaedic result values (OE),
- spacings (A1 A2),
- angles,
- coordinates of the reference points (R1, R2, R3) and
- a classification, and
- preferably further patient-specific information items, wherein, for this purpose, preferably a computer-readable file is generated which contains the information items in table form and/or contains the reference points (R1, R2, R3) as figures.

10. Device (6) for determining result values on the basis of a skeletal medical imaging recording (B), **characterised in that** the device comprises

- a data interface (7) for acquiring image data of a skeletal medical imaging recording (B),
- a reference point determining unit (8) configured for an automatic determination of reference points (R1, R2, R3) in the image recording (B),
- an output data interface (14) for the output of image data of a monitoring image (KB) to an image display unit (20),
- a reference value dependency determining unit (19) for determining a reference value dependency for a reference point (R1, R2, R3), which represents the dependency of an orthopaedic result value (OE) on a spatial displacement of the reference point (R1, R2, R3),
- a result value ascertaining unit (9) configured for a calculation of an orthopaedic result value (OE) based at least upon a spacing (A1, A2) between two reference points (R1, R2, R3) and/or upon an angle defined by reference points (R1, R2, R3).

11. Device (6) according to claim 10, comprising

- a displacement unit (22) that is configured for a manual displacement capability for the reference points (R1, R2, R3) in a monitoring image (KB),
- the reference value dependency determining unit (19) for determining a reference value dependency for the reference point (R1, R2, R3) together with a visualisation unit (19a) for visualising isolines (I) and/or a local acceptance range (AB) and/or class limits (KG).

12. Diagnostic station (17) for assessing a skeletal medical imaging recording (B), which is configured for carrying out a method according to one of the claims 1 to 9 and/or a device according to one of the claims 10 or 11.

13. Medical imaging system (1) comprising a device according to claim 10 or 11, in particular a diagnostic station (17) according to claim 12.

14. Computer program product having a computer program which is directly loadable into a storage apparatus of a computer system or a diagnostic station (17) of a medical imaging system (1), having program portions in order to carry out all the steps of the method according to one of the claims 1 to 9 when the computer program is executed in the computer system or in the diagnostic station (17).

15. Computer-readable medium on which program portions that can be read in and executed by a computer unit are stored, in order to carry out all the steps of a method according to one of the claims 1 to 9 when the program portions are executed by the computer unit.

**Revendications**

1. Procédé de détermination de valeurs de résultat sur la base d'une prise de vue (B) médicotechnique squelettique, **caractérisé par** les stades :

- on se procure une prise de vue (B) médicotechnique squelettique,
- on détermine automatiquement des points (R1, R2, R3) de référence dans la prise de vue (B),
- on affiche la prise de vue (B), ensemble avec les points (R1, R2, R3) de référence, dans le cadre d'une image (KB) de contrôle, et
- on détermine une dépendance de valeur de référence pour un point (R1, R2, R3) de référence, qui représente la dépendance d'une valeur (OE) de résultat orthopédique à un déplacement dans l'espace du point (R1, R2, R3) de référence,
- on calcule une valeur (OE) de résultat orthopédique sur la base d'au moins une distance (A1, A2) entre deux points (R1, R2, R3) de référence et/ou d'un angle défini par des points (R1, R2, R3) de référence.

2. Procédé suivant la revendication 1, comprenant les stades supplémentaires :

- on affiche la prise de vue (B), également ensemble avec des distances (A1, A2) et/ou des angles, dans le cadre de l'image (KB) de contrôle, et
- on met à disposition une possibilité manuelle de déplacement des points (R1, R2, R3) de référence dans l'image (KB) de contrôle.

3. Procédé suivant l'une des revendications précédentes, dans lequel on visualise la dépendance de valeur de référence ensemble avec le point (R1, R2, R3) de référence.

**4.** Procédé suivant la revendication 3, dans lequel, pour déterminer la dépendance de valeur de référence pour un point (R1, R2, R3) de référence

- on forme une valeur de résultat de base à partir d'un groupe de points (R1, R2, R3) de référence pertinents pour la valeur (OE) de résultat orthopédique concernée,
- on forme une valeur de résultat de comparaison à partir du groupe de points (R1, R2, R3) de référence pertinents pour la valeur (OE) de résultat orthopédique concernée, dans lequel le point (R1, R2, R3) de référence, pour lequel on détermine la dépendance de valeur de référence, est déplacé pour le calcul de la valeur de résultat de comparaison,

dans lequel on détermine de préférence une dépendance absolue de valeur de référence, à partir de la différence entre la valeur de résultat de comparaison et la valeur de résultat de base et, d'une manière particulièrement préférée, on en détermine une dépendance relative de valeur de référence à partir de la dépendance absolue de valeur de référence normée par la valeur de résultat de base, dans lequel on calcule la valeur de résultat de base et la valeur de résultat de comparaison, de préférence de la même façon que la valeur (OE) de résultat orthopédique.

**5.** Procédé suivant la revendication 3 ou 4, dans lequel, pour une dépendance de valeur de référence, notamment relative, pour un point (R1, R2, R3) de référence, on détermine des isolignes (I), qui caractérisent des positions, qui correspondent à une variation déterminée, de préférence en pourcentage, d'une valeur (OE) de résultat orthopédique,
et de préférence, on affiche ces isolignes (I) dans une image (KB) de contrôle.

**6.** Procédé suivant l'une des revendications 3 à 5, dans lequel, pour une dépendance de valeur de référence, notamment relative, pour un point (R1, R2, R3) de référence, on détermine un domaine (AB) local d'acceptation, dans laquelle un déplacement du point (R1, R2, R3) de référence provoque une variation relative de la valeur (OE) de résultat orthopédique dans un domaine d'acceptation global défini à l'avance,
et de préférence, on affiche ce domaine (AB) local d'acceptation dans une image (KB) de contrôle.

**7.** Procédé suivant l'une des revendications précédentes, dans lequel on effectue en outre un classement automatique d'au moins des parties de la prise de vue (B) médicotechnique squelettique, sur la base de la valeur (OE) de résultat orthopédique et/ou des coordonnées des points (R1, R2, R3) de référence au moyen de plages de norme, dans lequel le classement s'effectue de préférence à l'aide de valeurs limites, qui fixent les plages de norme,
dans lequel les valeurs limites se trouvent d'une manière particulièrement préférée dans une base de données.

**8.** Procédé suivant la revendication 7, dans lequel, pour une dépendance d'une valeur de référence, notamment relative, pour un point (R1, R2, R3) de référence, on détermine des limites (KG) de classe, dans lesquelles des déplacements du point (R1, R2, R3) de référence provoquent seulement des variations de la valeur (OE) de résultat orthopédique, qui sont dans un classement et, de préférence, on affiche ces limites (KG) de classe dans une image (KB) de contrôle.

**9.** Procédé suivant la revendication 7 ou 8, comprenant un établissement automatique d'un rapport contenant des informations choisies dans le groupe

- valeurs (OE) de résultat orthopédique,
- distances (A1, A2),
- angles,
- coordonnées des points (R1, R2, R3) de référence,
- un classement et
- de préférence d'autres informations spécifiques à un patient,
dans lequel on produit à cet effet, de préférence, un fichier déchiffrable par ordinateur, qui contient les informations sous forme de tables et/ou les points (R1, R2, R3) de référence sous la forme d'images.

**10.** Installation (6) de détermination de valeurs de résultat sur la base d'une prise de vue (B) médicotechnique squelettique, **caractérisée en ce que** l'installation comprend

- une interface (7) de données pour la saisie de données d'image d'une prise de vue (B) médicotechnique squelettique,

- une unité (8) de détermination de points de référence conçus pour une détermination automatique de points (R1, R2, R3) de référence dans la prise de vue (B),
- une interface (14) de données de sortie pour envoyer des données d'image d'une image (KB) de contrôle à une unité (20) d'affichage d'image,
- une unité (19) de détermination de dépendance de valeur de référence pour la détermination d'une dépendance de valeur de référence pour un point (R1, R2, R3) de référence, qui représente la dépendance d'une valeur (OE) de résultat orthopédique à un déplacement dans l'espace du point (R1, R2, R3) de référence,
- une unité (9) de détermination de valeur de résultat conçue pour un calcul d'une valeur (OE) de résultat orthopédique sur la base d'au moins une distance (A1, A2) entre deux points (R1, R2, R3) de référence et/ou d'un angle défini par des points (R1, R2, R3) de référence.

**11.** Installation (6) suivant la revendication 10, comprenant

- une unité (22) de déplacement conçue pour une possibilité manuelle de déplacement des points (R1, R2, R3) de référence dans une image (KB) de contrôle,
- l'unité (19) de détermination de dépendance de valeur de référence pour la détermination de la dépendance de valeur de référence pour le point (R1, R2, R3) de référence ensemble avec une unité (19a) de visualisation pour la visualisation d'isolignes (I) et/ou d'un domaine (AB) local d'acceptation et/ou de limites (KG) de classe.

**12.** Poste (17) de mise en évidence d'une prise de vue (B) médicotechnique squelettique, qui est conformé pour effectuer un procédé suivant l'une des revendications 1 à 9 et/ou qui comprend une installation suivant la revendication 10 ou 11.

**13.** Système (1) d'imagerie de la technique médicale comprenant une installation suivant la revendication 10 ou 11 et/ou un poste (17) suivant la revendication 12.

**14.** Produit de programme d'ordinateur comprenant un programme d'ordinateur, qui peut être chargé directement dans un dispositif de mise en mémoire d'un système informatique ou d'un poste (17) de mise en évidence d'un système (1) d'imagerie de la technique médicale, comprenant des parties de programme pour effectuer tous les stades du procédé suivant l'une des revendications 1 à 9, lorsque le programme d'ordinateur est réalisé dans le système informatique ou dans le poste (17) de mise en évidence.

**15.** Support, déchiffrable par ordinateur, sur lequel sont mises en mémoire des parties de programme déchiffrables et réalisables par une unité informatique pour effectuer tous les stades d'un procédé suivant l'une des revendications 1 à 9, lorsque les parties de programme sont réalisées par l'unité informatique.

FIG 1

EP 3 566 651 B1

FIG 2

FIG 3

FIG 4

# FIG 5

## FIG 6

FIG 7

EP 3 566 651 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2017196526 A1

- WO 2006000063 A1